Europäisches Patentamt

European Patent Office

Office européen des brevets

(19)

(11) Numéro de publication : **0 069 607 B1**

(12) **FASCICULE DE BREVET EUROPÉEN**

(45) Date de publication du fascicule du brevet :
06.05.87

(21) Numéro de dépôt : 82401035.9

(22) Date de dépôt : 08.06.82

(51) Int. Cl.⁴ : **F 16 B   7/10**, A 61 B   6/00,
A 61 B   6/02

(54) **Coulisseau porte-charge à structure télescopique, et installation de radiologie munie d'un tel coulisseau.**

(30) Priorité : 23.06.81 FR 8112297
12.03.82 FR 8204213

(43) Date de publication de la demande :
12.01.83 Bulletin 83/02

(45) Mention de la délivrance du brevet :
06.05.87 Bulletin 87/19

(84) Etats contractants désignés :
DE GB IT NL

(56) Documents cités :
EP-A- 0 015 612
AU-A-   489 148
DE-A- 1 466 883
DE-A- 2 120 344
FR-A- 1 071 714
FR-A- 1 407 471
FR-A- 1 490 958
FR-A- 1 572 841
FR-A- 2 264 363
GB-A-   916 178
GB-A- 2 056 830
GB-A- 2 086 700
US-A- 2 822 477
Document publicitaire de Siemens: End results
count: Siemens Imaging Systems"

(73) Titulaire : THOMSON-CSF
173, Boulevard Haussmann
F-75379 Paris Cedex 08 (FR)

(72) Inventeur : Caugant, Jean
THOMSON-CSF SCPI 173 bld Haussmann
F-75379 Paris Cedex 08 (FR)
Inventeur : Dale, Jacques
THOMSON-CSF SCPI 173 bld Haussmann
F-75379 Paris Cedex 08 (FR)

(74) Mandataire : Grynwald, Albert et al
THOMSON-CSF SCPI 19, avenue de Messine
F-75008 Paris (FR)

Jouve, 18, rue St-Denis, 75001 Paris, France

## Description

L'invention concerne un coulisseau porte-charge plus particulièrement destiné aux installations de radiologie. L'invention a plus particulièrement pour objet un tel coulisseau à structure télescopique présentant, au repos, un encombrement minimum tout en permettant de grandes amplitudes de mouvement de part et d'autre de la position de repos.

Une installation de radiologie comporte au moins une table d'examen sur laquelle le patient est placé, une source de rayonnement X et un récepteur d'image, le plus souvent un amplificateur de luminance. La source et le récepteur sont placés de part et d'autre de la table. Au cours d'un même examen, il est souvent nécessaire de changer l'incidence du rayonnement X par rapport au patient. La source et le récepteur doivent donc être mobiles par rapport à la table d'examen. Or, ces deux appareils sont lourds et volumineux. Leurs supports sont donc également la plupart du temps largement dimensionnés pour ne pas fléchir sous la charge.

Il est connu par la demande de brevet européen n° 0 015 612, une installation de radiologie dans laquelle une source de rayons X est déplacée le long d'une table d'examen, grâce à un agencement qui facilite l'accès au patient. Cette installation comprend un bâti télescopique permettant de déplacer l'ensemble d'une colonne, supportant la source à rayons X, le long de la table d'examen. Il en résulte un encombrement moindre qui améliore l'accès. Cependant, le bâti télescopique présente encore un encombrement important, même dans sa position repliée. D'autre part, les solutions mécaniques à rouleaux utilisées sont complexes et coûteuses. Il est à noter en outre que le déplacement de la source à rayons X est un déplacement linéaire qui n'est pas approprié pour l'angiographie, ou tout autre examen qui exige de placer correctement une source de rayons X et un récepteur par rapport à un centre d'analyse.

L'invention permet de disposer d'un ensemble plus compact et moins coûteux. Elle permet en outre une grande précision dans les mouvements rapides de tomographie. En effet, en tomographie, les vibrations de la source ou du récepteur en mouvement doivent être éliminés pour éviter de brouiller l'image formée sur le récepteur. Le coulisseau conforme à l'invention apporte une solution particulièrement satisfaisante à ce problème supplémentaire.

Plus précisément, l'invention concerne donc un coulisseau porte-charge pour une installation de radiologie du type comportant un premier support fixe, un second support mobile sur lequel ladite charge est fixée et un chariot médian par lequel lesdits supports sont accouplés, caractérisé en ce que lesdits supports et ledit chariot médian ont la forme générale de secteur de couronne, en ce que lesdits supports ont une section générale en forme de C et se font face par leurs ouvertures latérales tandis que ledit chariot a une section en forme générale de H dont les deux nervures parallèles sont respectivement engagées dans lesdites ouvertures latérales pour coopérer avec des gorges formant glissières desdits supports, en ce que des moyens d'actionnement dudit chariot par rapport audit premier support comportent un moteur muni d'une roue dentée et une première crémaillère en arc de cercle sur laquelle s'engrène ladite roue dentée et en ce que des moyens d'actionnement dudit second support par rapport audit chariot médian comportent un axe monté en rotation dans ledit chariot et portant une roue dentée à chacune de ses extrémités, une seconde crémaillère en arc de cercle portée par ledit premier support et une troisième crémaillère en arc de cercle parallèle à ladite seconde crémaillère et portée par ledit second support, lesdites roues dentées s'engrenant respectivement sur lesdites seconde et troisième crémaillères.

L'invention concerne aussi à titre d'application, une installation de radiologie utilisant de tels coulisseaux.

En effet, certains examens radiologiques du cœur nécessitent deux explorations sous des incidences différentes pour donner des vues plus exactes des cavités cardiaques et aussi pour permettre de déterminer leur volume. Ces examens sont pratiqués en injectant des produits de contraste dans le système sanguin. Ceux-ci sont assez mal supportés par l'organisme et peuvent même être à l'origine d'un arrêt cardiaque pendant l'examen, nécessitant des manœuvres de réanimation. On a donc intérêt, d'une part, à réduire au maximum la quantité de produit de contraste et, d'autre part, à disposer d'une installation de radiologie qui ne constitue pas une gêne pour l'équipe de réanimation et qui puisse de préférence être éloignée du patient en un temps très court.

Une demande de brevet britannique n° 2 056 830 décrit une installation comportant deux paires de source-récepteur et utilisable en angiographie. Un premier système comporte une source et un récepteur liés mécaniquement de sorte à pouvoir tourner autour d'un centre d'analyse. Un second système comprend une seconde source et un second récepteur mobiles chacun sur des rails dont l'un est au plafond et l'autre est au sol à côté de la table d'examen ; les rails étant parallèles à la table d'examen. L'inconvénient de cette disposition est que l'angle d'incidence que l'on peut obtenir à partir du second système est fixe, puisqu'il dépend de la position des rails, ce qui impose au médecin-radiologue une contrainte très importante. En outre les possibilités d'accès au patient ne sont pas favorables du fait, notamment, que dans le second système le chariot portant le second récepteur est situé à côté de la table et ne peut que gêner le radiologue.

On peut mentionner également que, dans le but de réduire la quantité de contraste injecté, on a déjà proposé une installation comportant deux systèmes source-récepteur agencés autour d'un centre d'analyse défini au voisinage de la table sur laquelle on dispose le patient, ces deux systèmes pouvant être mis en œuvre simultanément de façon à ne nécessiter qu'une seule injection de produit de contraste. Cette installation comporte principalement deux grands arceaux sensiblement en demi-cercle et chaque arceau porte à chacune de ses extrêmités une source de rayons X et un récepteur (à amplificateur de luminance), respectivement. Un rail de guidage est creusé le long de chaque arceau et un support lui-même mobile par rapport à la table est engrené dans ce rail. C'est donc l'ensemble de l'arceau, de la source et du récepteur qui décrit un mouvement circulaire lorsqu'on veut changer l'incidence de prise de vue. Ce type d'installation fonctionne de façon satisfaisante et permet notamment de limiter la dose de produit de contraste en exploration cardiovasculaire. Cependant, le volume des arceaux peut gêner l'accès au patient, c'est notamment particulièrement le cas de l'un deux qui est agencé avec un axe fictif de rotation orienté parallèlement à la table. D'autre part, les masses en porte-à-faux sont importantes et nécessitent des structures lourdes, au prix de revient élevé, de sorte que seuls les plus grands hôpitaux peuvent être équipés de telles installations (voir le document publicitaire de la Firme Siemens « End results count : Siemens Imaging Systems », mis à la disposition du public en mai 1982).

L'installation proposée, grâce à l'utilisation de plusieurs coulisseaux porte-charge décrits ci-dessus est plus simple et moins coûteuse que l'installation mettant en œuvre les deux arceaux. D'autre part, elle gêne le moins possible l'accès au patient et elle peut en outre être éloignée de la table en un temps très court en cas d'urgence.

Selon l'invention, ces avantages sont obtenus grâce à une installation de radiologie du type comportant deux systèmes source-récepteur agencés autour d'un centre d'analyse respectivement un système principal comprenant notamment un support à deux branches mobiles en rotation autour d'un axe horizontal, une source de rayonnement portée par l'une des branches et un récepteur correspondant porté par l'autre branche, ainsi qu'un système supplémentaire pour les examens nécessitant une vision simultanée d'une même région sous deux incidences différentes choisies, du type dans lequel ledit système supplémentaire comprend deux sous-ensembles mobiles et indépendants, respectivement inférieur et supérieur, portant chacun une source de rayonnement ou un récepteur, ou inversement, susceptibles d'être positionnés de part et d'autre dudit centre d'analyse, ladite installation étant caractérisée en ce que cette source et ce récepteur sont montés sur leurs sous-ensembles respectifs par l'intermédiaire de coulisseaux téléscopiques, respectifs, selon l'une des revendications précédentes, orientés pour leur permettre un déplacement dans des plans verticaux.

Ainsi, l'un des arceaux précités (celui qui s'étendait avec son axe fictif de rotation parallèle à celui de la table) est remplacé par deux sous-ensembles très mobiles, l'un se déplaçant, par exemple, le long d'une structure de guidage fixée au plafond de la salle d'examen et l'autre, sous forme d'un chariot roulant, venant se positionner sous la table d'examen.

L'invention sera mieux comprise et d'autres avantages de celle-ci apparaîtront mieux à la lumière de la description qui va suivre, donnée uniquement à titre d'exemple et faite en référence aux dessins annexés dans lesquels :

la figure 1 est une vue en perspective d'un coulisseau porte-charge selon l'invention ; et

la figure 2 est une vue générale schématique en perspective d'une installation de radiologie comportant des coulisseaux conformes à la figure 1.

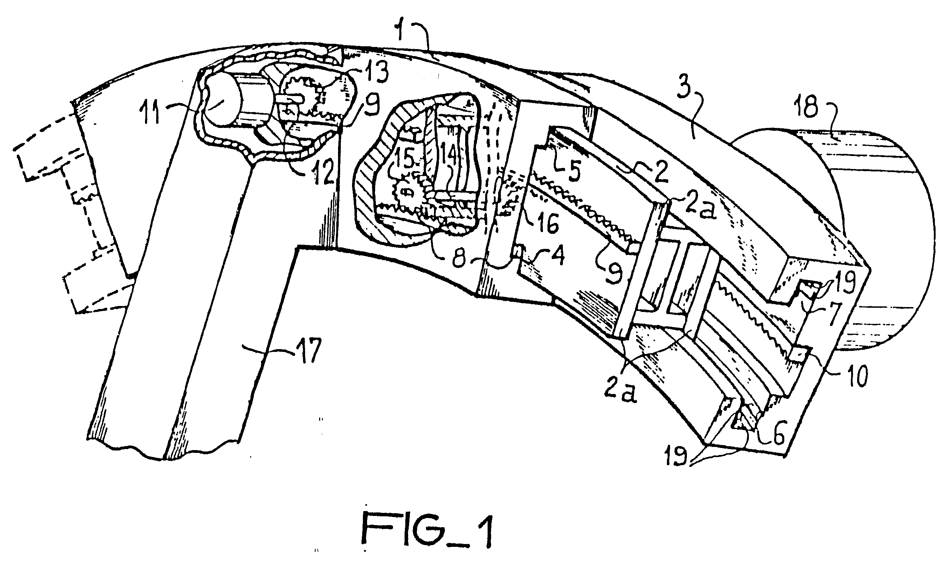

En référence à la figure 1, le coulisseau selon l'invention se compose principalement de trois éléments glissant les uns par rapport aux autres, à savoir un premier support 1, un second support 3 portant une charge 18, semblable au première support 1 et mobile de part et d'autre de celui-ci, ainsi qu'un chariot médian 2 assurant l'accouplement latéral desdits supports. Les supports et le chariot ont la forme générale de secteur de couronne. Les supports ont une section en forme générale de C tandis que le chariot a une section en forme générale de H. Ainsi, ces trois éléments sont accouplés latéralement et peuvent se déplacer les uns par rapport aux autres suivant des trajets circulaires parallèles à leur propre courbure. Les surfaces en regard creusées des supports 1 et 3 abritent le chariot médian 2, ce dernier assurant l'accouplement latéral des supports par la coopération des nervures 2a définies par le profil en H du chariot et des gorges formant glissières 4, 5 et 6, 7 définies par le profil en C des supports 1 et 3, respectivement. Chaque glissière est munie d'un ensemble de trois baguettes 19 facilitant le glissement du chariot par rapport au premier support d'une part et du second support par rapport au chariot d'autre part. Ces baguettes sont fabriquées en un matériau polymère organique du genre PTFE ou PYDANE. La charge 18 portée par le second support 3 peut être, en radiologie, une source de rayonnement X ou un récepteur à amplificateur de luminance.

Le système est complété par des moyens d'actionnement du chariot par rapport au premier support d'une part et par des moyens d'actionnement du second support par rapport au chariot d'autre part. Ces moyens d'actionnement sont mécaniquement couplés à un unique moteur d'entraînement 11 solidaire, dans l'exemple représenté, du premier support 1 et abrité à l'intérieur d'une colonne 17 maintenant l'ensemble à une certaine distance du patient. Les moyens d'entraînement du chariot 2 par rapport au premier support 1 comportent une première crémaillère 9, courbe, solidaire du chariot sur laquelle engrène une roue dentée 13 montée sur l'axe 12 du moteur 11. D'autre part, les moyens d'entraînement du second support 3 par rapport au chariot 2 comportent un axe 14 monté en rotation libre transversalement dans le chariot 2 et portant une roue dentée 15, 16, à chacune de ses extrêmités. La roue dentée 15 s'engrène sur une seconde crémaillère 8, courbe, du premier support 1 tandis que la roue dentée 16 s'engrène sur une troisième crémaillère 10, courbe, solidaire du second support 3 et parallèle à la seconde crémaillère 8. Les crémaillères 8 et 10 ont leurs dents en regard dans deux plans parallèles.

Le fonctionnement est le suivant : si on suppose que le moteur 11 tourne dans le sens des aiguilles d'une montre, la roue dentée 13 entraîne le chariot 2 vers la droite en considérant la figure 1 simultanément, l'ensemble constitué par l'axe 4 et les deux roues dentées 15 et 16 est entraîné en rotation par l'engrènement de la roue dentée 15 sur la crémaillère 8. Ainsi, la roue dentée 16 engrenée sur la crémaillère 10 qui est montée symétriquement par rapport à l'axe 14 relativement à la crémaillère 8 commande le déplacement du second support 3 par rapport au chariot 2, dans le même sens. Les roues 15 et 16 peuvent être choisies de même diamètre de façon que pour un déplacement donné du chariot par rapport au premier support, le second support se déplace d'une distance double.

Selon un autre mode de réalisation, les supports 1 et 3 et le chariot 2 peuvent être rectilignes.

Un avantage particulier du coulisseau qui vient d'être décrit est de permettre des déplacements rapides d'une charge lourde sans nécessiter la mise en mouvement de la colonne 17 elle-même. En radiologie en effet, il est connu que les mouvements d'angulation sont obtenus par rotation de la colonne autour d'un axe fixe. Le poids élevé de la charge oblige à ce que la colonne soit largement dimensionnée pour pourvoir supporter des accélérations élevées. Le coulisseau conforme à l'invention étant la seule partie mobile, on peut lui associer une colonne relativement légère. Il est possible de monter cette colonne 17 sur un chariot mobile, par exemple sur un rail horizontal.

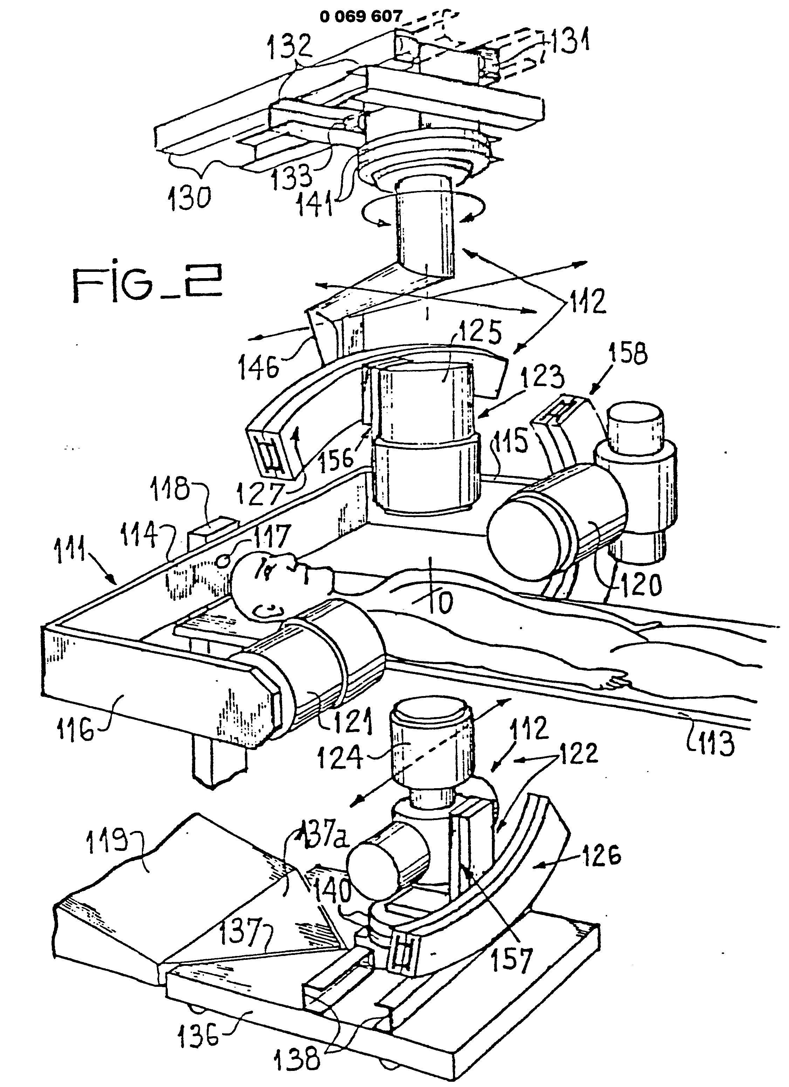

L'installation de radiologie représentée à la figure 2 constitue une application intéressante qui illustre bien les propriétés avantageuses du coulisseau qui vient d'être décrit. Elle se compose essentiellement de deux systèmes source-récepteur 111 et 112 agencés autour d'un centre d'analyse O défini à quelques centimètres au-dessus d'une table 113 sur laquelle le patient est allongé. Le système 111 qu'on appellera « système principal » comprend un support 114 à deux branches 115, 116 parallèles, mobile en rotation autour d'un axe horizontal 117 d'un support fixe 118 et reposant sur le sol au moyen d'un socle 119. La branche 115 porte à son extrémité une source de rayons X120 et la branche 116 porte à son extrémité un récepteur 121 à amplificateur de luminance. La source 120 et le récepteur 121 se font face de part et d'autre du centre d'analyse O, le système 112 qu'on appellera « système supplémentaire » est surtout destiné à être utilisé pour les examens nécessitant une vision simultanée d'une même région sous deux incidences différentes choisies, notamment les examens cardiovasculaires. Il se compose principalement de deux sous-ensembles 122, 123 mobiles et indépendants. Le sous-ensemble inférieur 122 porte une source de rayons X124 analogue à la source 120 et le sous-ensemble supérieur 123 porte un récepteur 125 analogue au récepteur 121. Cependant, il est possible d'inverser la situation, c'est-à-dire, de faire porter le récepteur 125 par le sous-ensemble 122 et la source 124 par le sous-ensemble 123. Cet agencement est avantageux pour l'examen des nourrissons car le récepteur 125 de volume relativement important par rapport à la taille de celui-ci est alors placé sous la table et ne gêne nullement le praticien pendant l'examen. Comme le montre clairement la figure 2, la source 124 et le récepteur 125 sont positionnés en vis-à-vis de part et d'autre du centre d'analyse O lorsque le système supplémentaire 112 est en position d'utilisation. Selon une caractéristique importante de l'invention, la source 124 et le récepteur 125 sont montés sur leurs sous-ensembles respectifs par l'intermédiaire de coulisseaux 126 et 127 en forme de secteur de couronne circulaire. Lorsque le sous-ensemble est en position d'utilisation, le centre de ces secteurs de couronne est confondu avec le centre d'analyse O précité. Ces coulisseaux sont conformes à la description qui précède. De cette façon, dans la position neutre de moindre encombrement illustrée à la figure 2, un coulisseau donné ne s'étend que sur une petite portion de couronne correspondant à un angle au centre de 30° alors que la source (ou le récepteur) peut se décaler de ± 30° par rapport à cette position centrale illustrée, lorsque ledit coulisseau télescopique arrive en fin de course d'un côté ou de l'autre de ladite position centrale.

Le sous-ensemble supérieur 123 est monté mobile le long de rails de guidage 130 fixés au plafond de la salle abritant l'installation, suivant une direction parallèle à celle de la table 113. Ces rails supportent les galets 131 d'un chariot intermédiaire défini principalement par deux petits rails 132 rectilignes, parallèles entre eux, s'étendant perpendiculairement aux rails 130 et le long desquels le reste du sous-ensemble 123 et assujetti à se déplacer sur une faible distance perpendiculaire à la table, grâce à des galets 133. Ce faible débattement permet d'aligner avec précision le récepteur 125 et l'organe à observer du patient, notamment le cœur.

Le sous-ensemble inférieur 122 est, quant à lui, installé sur un support déplaçable 136 tel qu'un chariot, roulant au sol et pourvu de moyens de positionnement et de blocage sous la forme d'une découpe en V, 137, coopérant avec des moyens complémentaires fixés au sol, par exemple une pointe 137a du socle 119. Le sous-ensemble 122 est monté sur son support déplaçable 136 par l'intermédiaire de deux autres petits rails 138 rectilignes et parallèles, le long desquels il est assujetti à se déplacer. Les rails 138 sont parallèles aux rails 132. Lorsque le support déplaçable est immobilisé en position d'utilisation telle que représentée. Bien entendu, les sous-ensembles 122 et 123 sont munis de moyens de blocage (non représentés) permettant de les immobiliser n'importe où le long des rails 132 et 138, respectivement, et on recherchera des positions maintenant la coaxialité des axes optiques de la source et du récepteur, soit par des moyens purement mécaniques, soit en prévoyant un couplage convenable entre deux moteurs (non représentés) assurant le déplacement des sous-ensembles le long des rails 132 et 138 correspondants. En revanche, les rails 130 sont essentiellement prévus pour amener le sous-ensemble 112 au-dessus du patient, des butées (non représentées) sont donc prévues pour l'immobiliser dans une position déterminée au-dessus de la table 113. On pourrait aussi imaginer de supprimer les rails 132 et d'orienter les rails 130 perpendiculairement par rapport à la position illustrée, le sous-ensemble 123 se déplaçant directement le long de ceux-ci avec une possibilité d'immobilisation en un emplacement quelconque au voisinage de la fin de course, pour l'alignement correct de la source et du récepteur.

D'autre part, chaque sous-ensemble 122, 123 est muni d'une articulation à axe vertical 140, 141, respectivement, par exemple sous la forme représentée de deux brides circulaires accouplées, tournant l'une par rapport à l'autre. L'articulation 140 du sous-ensemble 122 est prévue entre les rails 138 et le coulisseau 126 tandis que l'articulation 141 du sous-ensemble 123 est intercalée entre les rails 132 du chariot intermédiaire précité et un montant coudé 146 fixé au coulisseau 127. Dans les deux cas, la conformation géométrique des éléments de structure des sous-ensembles 122 et 123 est telle que l'axe de rotation de l'articulation 140 ou 141 soit confondu avec l'axe principal de symétrie de ladite source 124 ou dudit récepteur 125, respectivement, lorsque chaque coulisseau 126 ou 127 correspondant est dans sa partie centrale de moindre encombrement telle que représentée sur la figure 2, c'est-à-dire, la position pour laquelle les deux éléments externes du coulisseau considéré sont totalement en vis-à-vis l'un de l'autre. Par conséquent, la combinaison des mouvements de rotation au niveau des articulations 140 et 141 et de pivotement le long des coulisseaux 126 et 127, permet à la source 124 et au récepteur 125 d'explorer chacun un cône d'ouverture donné (ayant typiquement un angle d'ouverture de 60°) dont le sommet est confondu avec le centre d'analyse O. Bien entendu, une incidence choisie de la source à l'intérieur de son cône de déplacement entraîne automatiquement une orientation spécifique du récepteur, telle que celui-ci demeure en vis-à-vis de la source sensiblement axé le long d'une direction passant par le centre d'analyse. Pratiquement, ce résultat est obtenu en maintenant les coulisseaux 126 et 127 dans des plans parallèles et en donnant à ces coulisseaux des élongations curvilignes égales et suivant le même sens de rotation. Bien entendu, on peut prévoir dans ce but n'importe quel moyen d'accouplement connu, mécanique et/ou électronique, pour maintenir source et récepteur en position d'isocentrisme par rapport au centre d'analyse O.

D'autre part, la technique classique du grandissement radiologique nécessite de pouvoir déplacer le récepteur par rapport au patient le long de son axe d'alignement avec la source. Dans ce but, on voit que le récepteur 125 est monté sur son coulisseau 127 par l'intermédiaire d'un système de glissières rectilignes 156 agencé parallèlement à une direction radiale passant par le milieu du second support 147 (celui qui porte le récepteur) du coulisseau 127. Un système de glissières rectilignes 157 analogue au précédent, est prévu entre la source 124 et le coulisseau 126. Il trouve sa justification essentielle dans le fait, mentionné ci-dessus qu'on est parfois obligé d'inverser source et récepteur par rapport aux sous-ensembles 122 et 123, pour les examens en pédiatrie.

Enfin, il faut noter que la branche 115 du système principal 111 qui porte la source 120 est aussi équipée d'un coulisseau 158 analogue aux coulisseaux 126 et 127, intercalé entre ladite branche et ladite source. En radiologie cardiovasculaire, ce coulisseau sera maintenu dans sa position centrale d'élongation nulle, telle que représentée.

Cependant, pour certains types d'examens, notamment du crâne, il peut être utile de donner une incidence supplémentaire du faisceau de rayons X par rapport au récepteur. Le coulisseau 158 offre cette possibilité sans augmenter notablement l'encombrement et/ou le prix de l'installation.

Le fonctionnement de l'installation découle avec évidence de la description qui précède. Lorsqu'on veut pratiquer un examen sous deux incidences simultanées, il suffit d'amener le support déplaçable 136 portant la source de rayons X sous la table 113 pour le positionner en butée contre l'extrémité du socle 119 et de faire circuler le sous-ensemble 123 le long des rails 130 jusqu'à ce que le récepteur 125 soit en bonne position au-dessus du patient. On règle ensuite le bon alignement de la source et du récepteur par ajustement des positions respectives des sous-ensembles le long des rails 138 et 132, de façon que l'axe commun de la source et du récepteur passe par l'organe à examiner. Une incidence donnée est entièrement définie par rotations des articulations 140 et 141 (les coulisseaux 126 et 127 étant maintenus parallèles) et par élongation identique des coulisseaux.

Si une réanimation doit intervenir, le sousensemble 122 ne constitue aucune gêne pour le personnel tandis que le sous-ensemble 123 peut facilement être repoussé à bonne distance, le long des rails 130.

**Revendications**

1. Coulisseau porte-charge pour une installation de radiologie du type comportant un premier support fixe (1), un second support mobile (3) sur lequel ladite charge est fixée et un chariot médian (2) par lequel lesdits supports sont accouplés, caractérisé en ce que lesdits supports (1, 3) et ledit chariot médian (2) ont la forme générale de secteur de couronne, en ce que lesdits supports ont une section générale en forme de C et se font face par leurs ouvertures latérales tandis que ledit chariot a une section en forme générale de H dont les deux nervures parallèles (2a) sont respectivement engagées dans lesdites ouvertures latérales pour coopérer avec des gorges formant glissières (4, 5, 6, 7) desdits supports, en ce que des moyens d'actionnement dudit chariot par rapport audit premier support comportent un moteur muni d'une roue dentée et une première crémaillère (9) en arc de cercle sur laquelle s'engrène ladite roue dentée et en ce que des moyens d'actionnement dudit second support (3) par rapport audit chariot médian (2) comportent un axe (14) monté en rotation dans ledit chariot et portant une roue dentée (15, 16) à chacune de ses extrémités, une seconde crémaillère (8) en arc de cercle portée par ledit premier support et une troisième crémaillère (10) en arc de cercle parallèle à ladite seconde crémaillère et portée par ledit second support, lesdites roues dentées (15, 16) s'engrenant respectivement sur lesdites seconde et troisième crémaillères.

2. Coulisseau selon la revendication 1, caractérisé en ce que ledit moteur est porté par ledit premier support et ladite première crémaillère (9) est portée par ledit chariot médian.

3. Coulisseau selon la revendication 1 ou 2, caractérisé en ce que les gorges (4, 5, 6, 7) précitées sont pourvues de baguettes (19) formant glissières, par exemple en matériau polymère organique.

4. Installation de radiologie du type comportant deux systèmes source-récepteur (111, 112) agencés autour d'un centre d'analyse (O) respectivement un système principal (111) comprenant notamment un support à deux branches mobiles en rotation autour d'un axe horizontal (117), une source de rayonnement (120) portée par l'une des branches et un récepteur (121) correspondant porté par l'autre branche, ainsi qu'un système supplémentaire (112) pour les examens nécessitant une vision simultanée d'une même région sous deux incidences différentes choisies, du type dans lequel ledit système supplémentaire comprend deux sous-ensembles (122, 123) mobiles et indépendants, respectivement inférieur et supérieur, portant chacun une source de rayonnement (124) ou un récepteur (125), ou inversement, susceptibles d'être positionnés de part et d'autre dudit centre d'analyse, ladite installation étant caractérisée en ce que cette source et ce récepteur sont montés sur leurs sous-ensembles respectifs par l'intermédiaire de coulisseaux télescopiques (126, 127), respectifs, selon l'une des revendications précédentes, orientés pour leur permettre un déplacement dans des plans verticaux.

5. Installation selon la revendication 4, caractérisée en ce que chaque sous-ensemble est muni d'une articulation (140, 141) à axe vertical, pour permettre à ladite source ou audit récepteur lié à un coulisseau respectif précité d'occuper n'importe quelle position dans un volume cônique prédéterminé.

6. Installation selon la revendication 5, caractérisée en ce que ladite source et/ou ledit récepteur sont montés sur leurs coulisseaux respectifs par l'intermédiaire, chacun, d'un système de glissières rectilignes (156, 157) agencé parallèlement à une direction radiale passant par le milieu du coulisseau en secteur de couronne circulaire correspondant.

7. Installation selon l'une des revendications 4 à 6, caractérisée en ce que le sous-ensemble inférieur (122) précité est installé sur un chariot (136) roulant au sol, pourvu de moyens de positionnement et de blocage (137) coopérant avec des moyens complémentaires (137a) fixés au sol, par exemple combinés à un socle (119) dudit système principal.

8. Installation selon l'une des revendications 4 à 7, caractérisée en ce que le sous-ensemble supérieur (123) précité est monté mobile le long d'au moins un premier rail (130) ou analogue fixé par exemple au plafond de la salle abritant ladite installation.

9. Installation selon la revendication 8, caractérisée par un chariot intermédiaire mobile par rapport audit premier rail et comportant au moins un second rail (132) rectiligne, perpendiculaire audit premier rail et le long duquel ledit sous-ensemble supérieur est assujetti à se déplacer sur une certaine distance.

10. Installation selon l'ensemble des revendications 7 et 9, caractérisée en ce que ledit sous-ensemble inférieur est monté sur son chariot par l'intermédiaire d'au moins un troisième rail (138) rectiligne ou analogue, le long duquel il est assujetti à se déplacer, ledit troisième rail étant parallèle audit second rail lorsque ledit chariot est immobilisé en position d'utilisation.

11. Installation selon l'une des revendications 4 à 10, caractérisée en ce que la branche dudit système principal (115) qui porte la source de rayonnement (120) correspondante est équipée d'un coulisseau (158) selon l'une des revendications 1 à 3 sur lequel est montée ladite source.

**Claims**

1. Load-carrying slide of a radiologic unit, of the type comprising a first stationary carrier (1), a second, movable carrier (3) whereon said load is fixed, and a central carriage (2) whereby said carriers are coupled, characterized in that said carriers (1, 3) and said central carriage (2) have the general shapes of crown sectors, that said carriers have a general C-shaped section and face each other by their lateral openings while said carriage has a generally H-shaped section the two parallel legs (2a) of which are respectively engaged in said lateral openings to cooperate with grooves forming sliding guides (4, 5, 6, 7) of said carriers, in that means for actuation of said carriage with respect to said first carrier comprise a motor equipped with a gear and a first rack (9) shaped as a circular arc and engaged with said gear, and in that means for actuation of said second carrier (3) with respect to said central carriage (2) comprise an axis (14) rotatably mounted in said carriage and supporting a gear (15, 16) at each of its ends, a second rack (8) formed as a circular arc and supported by said first carrier and a third rack (10) formed as a circular arc and extending parallel to said second rack and supported by said second carrier, said gears (15, 16) being in meshing engagement with said second and third racks, respectively.

2. Slide according to claim 1, characterized in that said motor is supported by said first carrier and said first rack (9) is supported by said central carriage.

3. Slide according to claims 1 or 2, characterized in that the above-mentioned grooves (4, 5, 6, 7) are provided with guide rods (19), e. g. of an organic polymer material.

4. Radiologic unit of the type comprising two source-receiver systems (111, 112) arranged about an analyzing center (O), i. e. a main system (11) comprising, in particular, a carrier having two arms rotatably movable about a horizontal axis (117), a radiating source (120) supported by one of the arms and a corresponding receiver (121) supported by the other arm, as well as an additional system (112) for examinations requiring a simultaneous visualization of the same region under two different selected incidences, of the type wherein said additional system comprises two sub-sets (122, 123) which are movable and independent, i. e. a lower and an upper set, each carrying a radiation source (124) or a receiver (125), or vice versa, susceptible to be positioned on both sides of said analyzing center, said unit being characterized in that this source and this receiver are mounted on their respective sub-sets through telescopic respective slides (126, 127) according to any of the preceding claims, oriented in a manner to permit their movement in vertical planes.

5. Unit according to claim 4, characterized in that each sub-set is equipped with a joint (140, 141) having a vertical axis to permit said source or said receiver connected to a respective above-mentioned slide to assume any position in a conical predetermined volume.

6. Unit according to claim 5, characterized in that said source and/or said receiver are mounted on their respective slides through a rectilinear system of sliding guides (156, 157) each of which are arranged in parallel to a radial direction passing through the sector of the corresponding slide of circular crown sector shape.

7. Unit according to any of claims 4 to 6, characterized in that the above-mentioned lower sub-set (122) is mounted on a carriage (136) rolling on the ground and provided with positioning and blocking means (137) cooperating with complementary means (137a) fixed on the ground, e. g. combined with a base (119) of said main system.

8. Unit according to any of claims 4 to 7, characterized in that the above-mentioned upper sub-set (123) is mounted to be movable along at least one first rail (130) or a similar element which is e. g. secured on the ceiling of the room accommodating said unit.

9. Unit according to claim 8, characterized by an intermediate carriage which is movable with respect to said first rail and comprises at least a second rectilinear rail (132) which is perpendicular to said first rail and along which said upper sub-set is displaceable along a certain distance.

10. Unit according to the entirety of claims 7 and 9, characterized in that said lower sub-set is mounted on its carriage through at least one third rectilinear rail (138) or a similar member along which it is displaceable, said third rail being parallel to said second rail when said carriage is immobilized in its position of use.

11. Unit according to any of claims 4 to 10, characterized in that the arm of said main system (115) which carries the corresponding radiation source (120) is equipped with a slide (158) according to any of claims 1 to 3 and whereon said source is mounted.

**Patentansprüche**

1. Lasttragender Schieber für eine Radiologieanlage, vom Typ mit einem ersten festen Träger (1), einem zweiten, beweglichen Träger (3), worauf die Last befestigt ist, und einem mittleren Wagen (2), durch welchen die genannten Träger angekoppelt sind, dadurch gekennzeichnet, daß die genannten Träger (1, 3) und der mittlere Wagen (2) die allgemeine Form von Kranzsektoren aufweisen, daß die genannten Träger einen allgemein C-förmigen Querschnitt aufweisen und einander an ihren seitlichen Öffnungen zugewendet sind, während der Wagen einen allgemein H-förmigen Querschnitt aufweist, wobei die beiden parallelen Rippen (2a) des H jeweils in eine der seitlichen Öffnungen eingreifen, um mit Nuten zusammenzuwirken, die Schiebeführungen (4, 5, 6, 7) für die genannten Träger bilden, daß Mittel zur Verstellung des genannten Wagens bezüglich des ersten Trägers einen Motor umfassen, der mit einem Zahnrad und einer ersten Zahnstange (9) versehen ist, welche kreisbogenförmig ist und womit das genannte Zahnrad in Kämmeingriff steht, und daß Mittel zur Verstellung des zweiten Trägers (3) bezüglich des mittleren Wagens (2) eine in dem Wagen drehbeweglich gelagerte Achse (14), die ein Zahnrad (15, 16) an jedem ihrer Enden trägt, eine zweite kreisbogenförmige Zahnstange (8), welche der genannte erste Träger trägt, und eine dritte kreisbogenförmige Zahnstange (10) umfassen, welche parallel zu der genannten zweiten Zahnstange ist und von dem genannten zweiten Träger getragen wird, wobei die genannten Zahnräder (15, 16) mit der zweiten bzw. dritten Zahnstange in Kämmeingriff stehen.

2. Schieber nach Anspruch 1, dadurch gekennzeichnet, daß der genannte Motor von dem genannten ersten Träger getragen wird und die genannte erste Zahnstange (9) von dem genannten mittleren Wagen getragen wird.

3. Schieber nach Anspruch 1 oder 2, dadurch gekennzeichnet, daß die genannten Nuten (4, 5, 6, 7) mit Stäben (19) versehen sind, welche Schiebebahnen bilden, beispielsweise aus organischem Polymermaterial.

4. Radiologieanlage vom Typ mit zwei Quelle/Empfänger-Systemen (111, 112), welche um ein Analysezentrum (O) herum angeordnet sind, nämlich ein Hauptsystem (111), das insbesondere einen Träger mit zwei Armen aufweist, die drehbeweglich um eine waagerechte Achse (117) sind, eine von einem der Arme getragene Strahlungsquelle (120) aufweist und einen entsprechenden, vom anderen Arm getragenen Empfänger (121) besitzt, sowie mit einem Nebensystem (112) für Untersuchungen, bei denen eine gleichzeitige Betrachtung desselben Bereiches unter zwei verschiedenen gewählten Einfallsrichtungen benötigt wird, vom Typ bei welchem das Nebensystem zwei Untergruppen (122, 123) umfaßt, die beweglich und voneinander unabhängig sind, nämlich ein unteres und ein oberes, welche jeweils eine Strahlungsquelle (124) oder einen Empfänger (125) tragen oder umgekehrt, die auf der einen bzw. anderen Seite des Analysezentrums angeordnet werden sollen, wobei diese Anlage dadurch gekennzeichnet ist, daß diese Quelle und dieser Empfänger auf ihren zugehörigen Untergruppen jeweils über zugehörige Teleskopschieber (126, 127) nach einem der vorstehenden Ansprüche gelagert sind, welche dergestalt orientiert sind, daß sie eine Bewegung in Vertikalebenen ausführen können.

5. Anlage nach Anspruch 4, dadurch gekennzeichnet, daß jede Untergruppe mit einem eine senkrechte Achse aufweisenden Gelenk (140, 141) versehen ist, damit die genannte Quelle bzw. der genannte Empfänger, der mit einem entsprechenden genannten Schieber verbunden ist, jedwede Position in einem vorbestimmten konischen Volumen einnehmen kann.

6. Anlage nach Anspruch 5, dadurch gekennzeichnet, daß die genannte Quelle und/oder der genannte Empfänger auf ihren zugehörigen Schiebern jeweils über ein Geradführungssystem (156, 157) angebracht sind, das parallel zu einer Radialrichtung angeordnet ist, welche durch den Mittelpunkt des entsprechenden rundkranzsektorförmigen Schiebers verläuft.

7. Anlage nach einem der Ansprüche 4 bis 6, dadurch gekennzeichnet, daß die untere Untergruppe (122) auf einem auf dem Boden verfahrbaren Wagen (136) aufgebaut ist, welcher mit Positionier- und Blockiermitteln (137) versehen ist, die mit komplementären, am Boden befestigten Mitteln (137a) zusammenwirken, die beispielsweise mit einem Sockel (119) des Hauptsystems kombiniert sind.

8. Anlage nach einem der Ansprüche 4 bis 7, dadurch gekennzeichnet, daß die obere Untergruppe (123) beweglich gelagert ist entlang wenigstens einer Schiene (130) oder dergleichen, die beispielsweise an der Decke des Raumes angebracht ist, welcher die genannte Anlage aufnimmt.

9. Anlage nach Anspruch 8, gekennzeichnet durch einen Zwischenwagen, welcher bezüglich der genannten ersten Schiene beweglich ist und wenigstens eine zweite geradlinige Schiene (132) umfaßt, die senkrecht zu der ersten Schiene ist und entlang welcher die genannte obere Untergruppe über eine bestimmte Strecke bewegbar ist.

10. Anlage nach der Gesamtheit der Ansprüche 7 und 9, dadurch gekennzeichnet, daß die genannte untere Untergruppe auf ihrem Wagen über wenigstens eine dritte Schiene (138) gelagert ist, die geradlinig oder in ähnlicher Weise verläuft und entlang welcher er bewegbar ist, wobei diese dritte Schiene parallel zu der zweiten Schiene ist, wenn der genannte Wagen in der Gebrauchsstellung festgelegt ist.

11. Anlage nach einem der Ansprüche 4 bis 10, dadurch gekennzeichnet, daß derjenige Arm des Hauptsystems (115), welcher die entsprechende Strahlungsquelle (120) trägt, mit einem Schieber (158) nach einem der Ansprüche 1 bis 3 ausgestattet ist, woran die genannte Quelle angebracht ist.

FIG_1

0 069 607

FIG_2

132
131
130
133
141
112
146
125
123
158
115
156
127
118
114
117
111
120
121
124
112
116
122
119
137a
126
140
157
137
113
0
136
138